# EUROPEAN PATENT APPLICATION

(11) **EP 4 309 567 A1**
(43) Date of publication of application: **24.01.2024**
(21) Application number: 22185651.1
(22) Date of filing: 19.07.2022
(51) Int. Cl.: A61B 5/00, A61B 5/107

(54) **METHOD AND SYSTEM FOR PROCESSING A PLURALITY OF CAMERA IMAGES OF A HUMAN BODY TARGET AREA OVER TIME**

(71) Applicant: Universiteit Antwerpen, 2000 Antwerpen (BE)
(72) Inventor: VANLANDUIT, Steve, 2020 Antwerpen (BE); RIBBENS, Bart, 2020 Antwerpen (BE); SELS, Seppe, 2020 Antwerpen (BE)
(74) Representative: IP HILLS NV

(57) **Abstract**

A method and a system for processing a plurality of camera images of a human body target area over time, the method comprising the steps of obtaining a plurality of images of a human body target area of a human body captured by a first camera over time; obtaining physical data of said human body; and generating a 3D model of at least said human body target area.

## Description

### Field of the Invention

The present invention generally relates to a method and a system for processing a plurality of camera images of a human body target area over time.

### Background of the Invention

Thermal imaging of a human body is configured to record thermal radiation of the human body. It is known in the art that thermal imaging or thermography of a human body can contribute to the detection and/or follow-up of lesions or of various abnormal body conditions. As an example, cancerous tissue may be recognized in thermal imaging due to increased metabolic activity and blood flow in and around the affected tissue. Another example is tissue affected by an inflammation or infection, which may also be detected due to increased thermal radiation. Additionally, in sports medicine, a relatively high temperature of a part of the human body can point to a potentially undetected physical overloading. Thermal imaging, as well as imaging in other wavelengths such as UV or visual light, of a human body may thus be advantageous for monitoring a condition of a target area of the human body over time in various medical disciplines.

A known problem of thermography is however the relatively limited resolution of thermal imaging cameras or infrared cameras. Moreover, thermal imaging can only provide images of a human body at a given moment in time, but the comparison of images taken at different moments may be relatively complicated because the mapping of said images to a determined anatomical body part or anatomical target area may prove to be relatively difficult. Even if a patient may be positioned in a substantially identical position for each image, the patient may have taken or lost weight, for example when images are taken once a month, and the patient may thus have changed shape, which may hamper identification of a same target area in images taken at different moments in time. When a patient is moving during thermography, which may for example be of use in sports medicine, identification of a human body target area to be followed up in a temporal series of images over time, may still be more difficult, in particular, when a single two-dimensional (2D) imaging camera is used.

### Summary of the Invention

It is therefore an aim of the present invention to solve or at least alleviate one or more of the above-mentioned problems. In particular, the invention aims at providing a computer-implemented method and system for processing a plurality of camera images of a human body target area over time such that monitoring a condition of said human body target area over time becomes possible.

To this aim, according to a first aspect of the invention, there is provided a computer-implemented method for processing a plurality of camera images of a human body target area over time having the features of claim 1. In particular, the method comprises the steps of obtaining a plurality of images of a human body target area of a human body captured by a first camera over time. A human body target area can for example be a shoulder, or part of a chest area or a knee, or any other part of a human body. The first camera is configured to capture images in a predetermined range of wavelengths of the electromagnetic wave spectrum. Medical imaging devices based on X-ray emission, computed tomography scans or on nuclear magnetic resonance imaging are not considered to be cameras. The method further comprises the steps of obtaining physical data of said human body. As an example, said physical data may include measuring data, such as one or more of a length of the human body, a weight of said human body, a circumference of a chest or a circumference of a hip of said human body or any other relevant body measure. Said physical data may further include one or more of an age or a gender of said human body. The method further comprises the step of generating a three-dimensional (3D) model of at least said human body target area, more preferably of said substantially entire human body including said human body target area, based on a statistical shape model (SSM) of a human body and based on said obtained physical data of said human body. The statistical shape model is a generic function allowing to represent a plurality of specific human bodies including as many relatively common potential deformations as possible of said human bodies based on a set of parameters which are specific for a given human body. Background and examples of such statistical shape models have been described in literature, for example in Danckaers, F., Huysmans, T., Lacko, D., & Sijbers, J. (2015), Evaluation of 3D Body Shape Predictions Based on Features, 258-265 (https://doi.org/10.15221/15.258), or in Danckaers, F., Huysmans, T., Lacko, D., Ledda, A., Verwulgen, S., van Dongen, S., & Sijbers, J. (2014), Correspondence Preserving Elastic Surface Registration with Shape Model Prior, 2014, 22nd International Conference on Pattern Recognition (https://doi.org/10.1109/ICPR.2014.373) or in Amberg, B., Romdhani, S., & Vetter, T. (n.d.), Optimal Step Nonrigid ICP Algorithms for Surface Registration in Shape Model Prior, 2014, 22nd International Conference on Pattern Recognition (https://doi.org/10.1109/ICPR.2014.373). The generated SSM-based 3D-model, which is specific for said given human body at a given moment in time, includes a plurality of landmark points distributed over a boundary surface of the model such that each landmark point always refers to the same anatomical location in each of the generated 3D models. As an example, a human body may have gained or lost weight over time, for example over months, provoking a change in shape or a deformation of the human body target area. The statistical shape model is configured to account for such deformations such that a predetermined anatomical location is linked to the same landmark point in each of the generated SSM-based 3D models for each of these moments in time. The method further comprises the step of mapping said plurality of images on the generated SSM-based 3D-model of said human body target area, thereby obtaining an anatomical alignment of said plurality of images of said human body target area. As a result of this improved alignment of images of said human body target area, it is possible to monitor said human body target area over time. Monitoring over time is understood as providing measurement values, in particular of a specific location of a human body target area, at various discrete points in a time interval, which is more information than just providing a single value at one time instance or a mean value over a given time span. Thanks to the present method, monitoring of a human body target area may be done during movement of said human body target area or may be done over a relatively large time interval such as over months or a year or more or less. From the monitoring data over time of said human body target area, medical information as to the state of the human body may be deduced.

The first camera can preferably be one of an infrared camera, a UV camera or a visual light camera. A visual light camera, as well as a UV camera, are configured to capture a reflection of visual light, or UV respectively, by a surface, in particular, by the surface of a human body. An infrared camera is configured to capture IR emission by a body, for example by the human body. This is different to for example X-ray detectors which detect an attenuation of X-rays emitted by an X-ray emitter and going through the human body, which are therefore not considered as cameras. As an example, a temperature or a thermal condition of said human body target area can be monitored over time relatively accurately when using an infrared camera. Monitoring data of a thermal condition of said human body target area over time may for example provide information on an inflammation, on blood circulation, or on an overload of said human body target area. Since a resolution of an infrared camera may not be sufficient to align a plurality of images of a moving subject over time directly, the mapping of said plurality of images on the generated SSM-based 3D model allows one to obtain said anatomical alignment of said plurality of images of said human body target area. Additionally, and/or alternatively, a UV camera may for example be used to an advantage in the monitoring of burns, or a visual light camera may be used in the monitoring of melanoma.

Said first camera may advantageously be configured to capture 2D images of said human body target area. Obtaining 2D images and processing 2D images requires relatively low computing capacity and can therefore allow relatively fast image processing. Thanks to the mapping on the SSM-based 3D model, the first camera need not provide extremely precise images, i.e. images having a relatively high resolution. A relatively rough image quality, for example due to a relatively low image resolution, can still allow a relatively precise image alignment. A camera configured to provide 2D images can also be relatively cost-efficient.

It is preferred that the method further comprises the step of obtaining a plurality of visual images of at least said human body target area captured by a second camera over time. The capturing of said plurality of images by said second camera is done substantially simultaneously with the capturing of said plurality of images by said first camera. The second camera can preferably be configured to obtain a plurality of images of substantially the entire human body while the first camera is configured to obtain images of the human body target area. In other words, the first camera can be configured to zoom in on a specific part of the human body while the second camera may be configured to provide substantially whole-body images. Additionally, and/or alternatively, the second camera may be particularly useful when a spatial resolution of the first camera is relatively low. In this way, the plurality of visual images can help in determining a relatively exact position of the human body over time, which information may serve as input to adjust the statistical shape model of said human body target area.

The second camera may for example be an RGB camera. Such cameras can provide relatively precise images, in particular of moving human body target areas. Additionally, and/or alternatively, the second camera may be configured to capture 2D images, thus requiring less processing capacity than for example 3D images or stereoscopic images.

The method can preferably further comprise the step of skeletonizing the obtained visual images of said human body target area, or more preferably of said substantially entire human body. Skeletonizing is a process for reducing foreground regions in a binary image, which can be extracted from visual images using segmentation, to a skeletal remnant that largely preserves the extent and connectivity of the original region while throwing away most of the original foreground pixels. In other words, images of the human body can be reduced to a plurality of connected and/or hinged lines which can correspond more or less to a simplified human skeleton. In this way, movement of the human body, of which images of a human body target area are being obtained, can be simulated relatively well while keeping processing capacity requirements relatively low.

It is preferred that the mapping of said plurality of images on the generated SSM-based 3D model is based on the obtained plurality of visual images, in particular the skeletonized visual images. In other words, the plurality of obtained visual images can help in determining a pose of the human body at a given moment in time. In this way, the mapping of said plurality of images on the generated 3D model can then take into account said particular pose of the human body for said given moment in time, which can allow adjustment of an alignment of the plurality of images on the generated 3D model.

The method can preferably further include the step of warping the generated 3D model of at least said human body target area, more preferably of substantially the entire human body. Warping is understood as a deformation of the generated 3D model including bending, twisting, curving etc without changing any dimensions of the 3D model. By warping the generated 3D model, the model can imitate any pose or position of at least the human body target area or of substantially the entire human body, which can improve the mapping of the captured plurality of images on the generated 3D model, and therefore improve the anatomical alignment of said plurality of images.

The warping of the generated 3D model can advantageously be based on said obtained plurality of visual images, preferably of said skeletonized visual images, of the human body target area, and more preferably on the plurality of skeletonized visual images of substantially the entire human body. In other words, the generated 3D model of at least the human body target area, or preferably of the human body, can be warped into a substantially identical position or pose as a pose derived from one of the plurality of skeletonized visual images of said human body captured at a predetermined moment in time. As a result, a time series of warped SSM-based 3D models is obtained representing for example a movement of the human body including the human body target area over time. The mapping of said plurality of images captured by the first camera may then be performed on the plurality of warped SSM-based 3D models, in particular on the generated 3D model warped on the basis of the visual image taken at substantially the same time as the respective image of the plurality of images captured by the first camera.

The method can preferably further comprise the step of estimating a relative position, including for example an angle and/or a distance, of said first camera with respect to said second camera based on a virtual image of the generated SSM-based 3D model of the human body target area. Since the warping of the generated 3D model is preferably based on the skeletonized visual images captured by the second camera, an angle between said generated 3D model and the second camera is known. Since an angle between said first camera and said second camera may not always be known for every pair of images captured substantially simultaneously by the first, respectively, the second camera, in particular with images captured over a relatively long period of time, an adjustment algorithm may be used in which a virtual camera can make a virtual image of said generated 3D model in a simulation environment. Both the virtual image as well as the image captured by the first camera can be binarized. In an iterative way, said generated 3D model may then be slightly shifted, and the virtual image may be updated until the binarized virtual and first camera images are substantially similar, indicating that said virtual image aligns with a foreground of the respective image captured by the first camera, thus providing a relative position of the first camera with respect to the second camera. In this way, the position of the generated 3D model may be finetuned with respect to the first camera such that the mapping of said plurality of images of the human body target area captured by said first camera on the generated 3D model can also be finetuned, thus improving the anatomical alignment of the images.

According to a further aspect of the invention, there is provided a system for processing a plurality of camera images of a human body target area over time having the features of claim 11. In particular, the system comprises a first camera configured to capture a plurality of images of a human body target area over time, and a controller and a memory with computer program code configured to perform the method as described above. Such a system can provide one or more of the above-mentioned advantages. In particular, the system is a relatively low-cost system which can allow obtaining an anatomical alignment of said plurality of images of said human body target area, such that a relatively accurate monitoring over time of said human body target area, in particular, of a specific location within said human body target area, is possible.

The system can preferably further comprise a second camera configured to capture a plurality of visual images of a human body target area over time substantially simultaneously with the capturing of images by the first camera. The first camera can for example be configured to capture 2D images, for example infrared images of a human body target area, while the second camera can be configured to capture 2D visual images of substantially the entire human body including the human body target area.

According to a further aspect of the invention, there is provided a controller comprising at least one processor and at least one memory including computer program code, the at least one memory and computer program code configured to, with the at least one processor, cause the controller to perform the method as described above.

According to a further aspect of the invention, there is provided a computer program product comprising computer-executable instructions for performing the method as described above when the program is run on a computer.

According to a further aspect of the invention, there is provided a computer readable storage medium comprising computer-executable instructions for performing the method as described above the program is run on a computer.

### Brief Description of the Drawings

Fig. 1 shows a first embodiment of a system for processing a plurality of camera images of a human body target area over time according to an aspect of the invention;
Fig. 2 shows a schematic graph of a preferred embodiment of the computer-implemented method for processing a plurality of camera images of a human body target area over time according to an aspect of the invention;
Fig. 3 shows a result of the preferred embodiment of the computer-implemented method schematically shown in Figure 2; and
Fig. 4 shows a computing system suitable for performing various steps of the method for processing a plurality of camera images of a human body target area over time according to an aspect of the invention.

### Detailed Description of Embodiment(s)

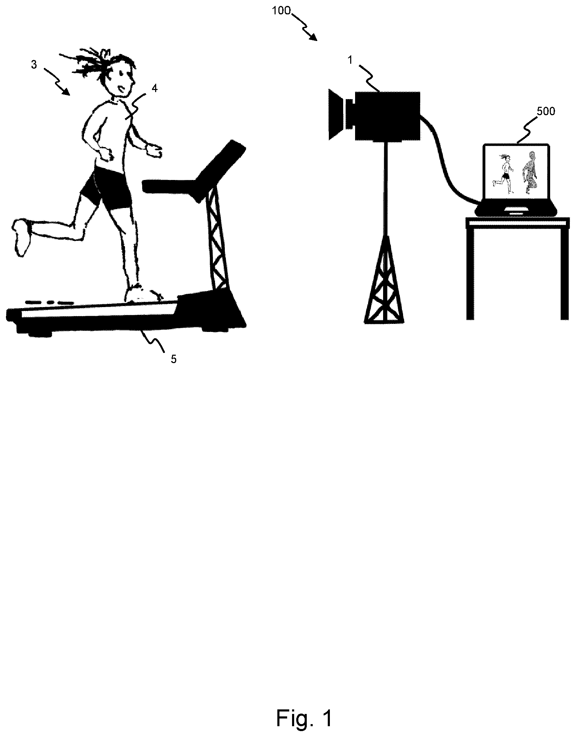

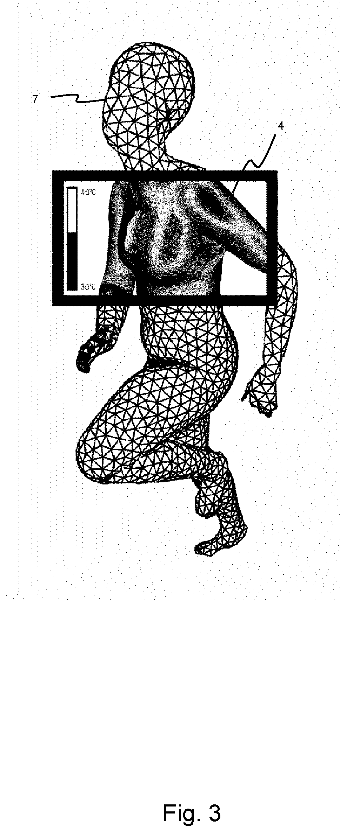

Figure 1 shows a first embodiment of a system 100 for processing a plurality of camera images of a human body target area over time. The system 100 may for example be used in sports medicine where monitoring of a human body 3 over time while doing sports may be desirable, for example to study overload of a human body target area 4, such as muscles, joints or organs, or to follow up an inflammation of one of said human body target areas. The patient may for example be running on a treadmill 5 for the monitoring, but many other set-ups are possible as well, such as for example cycling on a stationary bike or exercising on a training device or lifting weights or just jumping or doing other exercises without using any additional training device. The system 100 includes a first camera 1 configured to capture a plurality of images of a human body target area over time. The first camera may for example be configured to capture a plurality of images of a chest of the human body 3 while the patient is doing exercises, for example running, on the treadmill 5. The first camera 1 is preferably a 2D camera configured to capture 2D images of the human body target area, which require relatively low computing capacity to process. The first camera 1 is preferably an infrared camera configured to capture infrared images of said human body target area 4 since infrared or thermal imaging can show thermal information of the human body target area which may be indicative of a state of blood circulation and/or of potential overload and/or of any inflammation of (part of) said target area. Since these elements may vary during physical exercise, a monitoring over time during physical exercise may be appropriate. 'Over time' may mean during an uninterrupted period of time, for example over a time span of 5 minutes or more or less. Additionally, and/or alternatively, 'over time' may mean at regular or irregular intervals, for example once every week, or a number of times over a year. However, given the generally relatively low resolution of infrared cameras, it may be relatively difficult to correctly align images captured over time by the first camera 1, which is needed when comparison of said images is desired, for example to compare a status of an inflammation of said human body target area over time. When images captured over a relatively long period of time, such as months or years, have to be compared, there is an additional difficulty: the human body target area 4 may have changed shape which can highly complicate or even render impossible a correct anatomical alignment of captured infrared images, i.e. without a correct alignment of the images with respect to the anatomy of said human body target area. Without correct anatomical alignment, monitoring over time may be strongly hampered. Therefore, the system 100 further comprises a computer system 500, as shown for example in Figure 3, including a controller and a memory with computer program code configured to perform the computer-implemented method as will be described hereafter.

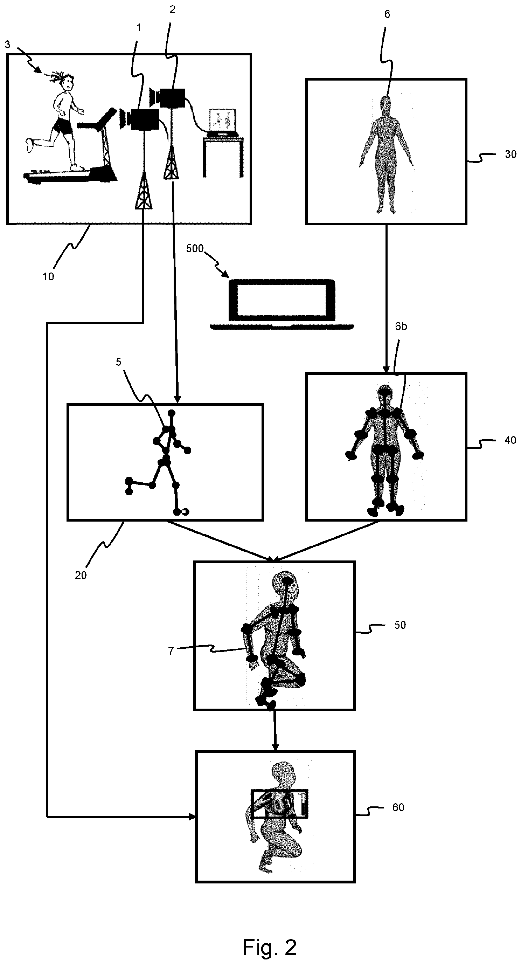

Fig. 2 shows a schematic graph of a preferred embodiment of the computer-implemented method for processing a plurality of camera images of a human body target area 4 over time. In addition to the system 100 as shown in Figure 1, the system can include a second camera 2. Said second camera 2 may for example be an RGB camera configured to capture a plurality of visual images of the human body target area 4 over time, or more preferably of substantially the entire human body 3 including the human body target area 4. The second camera 2 can preferably also be configured to capture 2D images. The first camera 1 and the second camera 2 are configured to capture said respective plurality of images over time substantially simultaneously. The first camera 1 can for example be an infrared camera focussing only on the human body target area 4, for example on an upper chest area, while the second camera 2 may capture images of substantially the entire human body 3.

In step 10 of a preferred embodiment of the method, the plurality of images captured by the first camera 1 and by the second camera 2 over time are obtained by a computing system 500. In step 20, a visual image captured at a predetermined time t by the second camera 2, preferably of substantially the entire human body 3, may then be processed by applying skeletonization, which is a process for reducing foreground regions in a binary image, obtained by segmenting said visual image captured by the second camera 2, to a skeletal remnant that largely preserves the extent and connectivity of the original region while throwing away most of the original foreground pixels. As a result, a skeletonized model 5 of a momentary posture of the human body 3 at time t is obtained.

In a parallel or consecutive step 30, physical data of said human body 3 are obtained by the computer system 500. The physical data may include one or more of a length of the human body 3, a weight of said human body 3, a circumference of a chest or a circumference of a hip of said human body 3 or any other relevant body measure. Said physical data may further include an age of the patient and a gender indication. Said physical data are then used by the computer system 500 to generate a 3D model 6 of at least said human body target area 4, but more preferably of substantially the entire human body 3. The generation of said 3D model 6 is based on a statistical shape model (SSM) of a human body, which is a generic function allowing to represent a plurality of specific human bodies including as many relatively common potential deformations as possible of said human bodies. The 3D model 6 includes a plurality of landmark points distributed over a boundary surface of the model such that each landmark point always refers to the same anatomical location in each of the generated 3D models. In a next step 40, said generated 3D model may also be skeletonized 6b in a way similar to the skeletonizing in step 20.

In a next step 50, the skeletonized model 5 of a momentary posture of the human body 3 at time t can be combined with the generated, and optionally skeletonized, 3D model 6, respectively 6b, to warp the generated 3D model into substantially the same posture as the skeletonized model at time t, and thus as the human body 3 at time t. Since the skeletonizing of step 20 is preferably repeated for the plurality of images captured by the second camera 2, step 50 can result in a plurality of warped generated 3D models 7 representing a movement and/or an evolution of the human body 3 over a predetermined window of time.

In a final step 60, the plurality of images captured by the first camera 1, in particular, the images, such as 2D infrared images, of the human body target area 4, for example a chest area, are mapped onto the generated SSM-based 3D model of said human body target area 4, or more preferably of said human body 3 including said human body target area 4, as shown in more detail in Figure 3 showing a thermal image of a chest area being mapped onto the generated and warped 3D model 7 for a given posture of the human body 3 at a predetermined time t. Since the respective anatomical positions of the human body 3 are always linked to the same respective landmark points in the generated 3D model thanks to the dedicated statistical shape model, an anatomical alignment of said plurality of images of said human body target area is obtained. In other words, the method can assure that the alignment of images is anatomically correct. Such an alignment can then allow to compare the plurality of images of the human body target area 4 over time in spite of a potential lack of resolution, for example for infrared images. The mapping and alignment of images can still be improved by correcting for the angle of view between the first camera 1 and the second camera 2 during the mapping step 60, for example by using an adjustment algorithm based on a virtual image of the warped 3D model 7, as explained before.

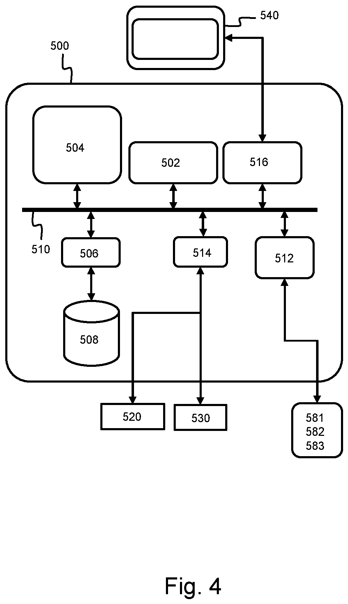

Figure 4 shows a suitable computing system 500 comprising circuitry enabling the performance of steps of embodiments of the method for processing a plurality of camera images of a human body target area over time according to an aspect of the invention. Computing system 500 may in general be formed as a suitable general-purpose computer and comprise a bus 510, a processor 502, a local memory 504, one or more optional input interfaces 514, one or more optional output interfaces 516, a communication interface 512, a storage element interface 506, and one or more storage elements 508. Bus 510 may comprise one or more conductors that permit communication among the components of the computing system 500. Processor 502 may include any type of conventional processor or microprocessor that interprets and executes programming instructions. Local memory 504 may include a random-access memory (RAM) or another type of dynamic storage device that stores information and instructions for execution by processor 502 and/or a read only memory (ROM) or another type of static storage device that stores static information and instructions for use by processor 502. Input interface 514 may comprise one or more conventional mechanisms that permit an operator or user to input information to the computing device 500, such as a keyboard 520, a mouse 530, a pen, voice recognition and/or biometric mechanisms, a camera, etc. Output interface 516 may comprise one or more conventional mechanisms that output information to the operator or user, such as a display 540, etc. Communication interface 512 may comprise any transceiver-like mechanism such as for example one or more Ethernet interfaces that enables computing system 500 to communicate with other devices and/or systems, for example with other computing devices 581, 582, 583. The communication interface 512 of computing system 500 may be connected to such another computing system by means of a local area network (LAN) or a wide area network (WAN) such as for example the internet. Storage element interface 506 may comprise a storage interface such as for example a Serial Advanced Technology Attachment (SATA) interface or a Small Computer System Interface (SCSI) for connecting bus 510 to one or more storage elements 508, such as one or more local disks, for example SATA disk drives, and control the reading and writing of data to and/or from these storage elements 508. Although the storage element(s) 508 above is/are described as a local disk, in general any other suitable computer-readable media such as a removable magnetic disk, optical storage media such as a CD or DVD, -ROM disk, solid state drives, flash memory cards, ... could be used.

As used in this application, the term "circuitry" may refer to one or more or all of the following:
(a) hardware-only circuit implementations such as implementations in only analog and/or digital circuitry and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions) and
(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented method for processing a plurality of camera images of a human body target area over time, the method comprising the steps of:
- obtaining a plurality of images of a human body target area of a human body captured by a first camera over time;
- obtaining physical data of said human body;
- generating a three-dimensional (3D) model of at least said human body target area based on a statistical shape model (SSM) of a human body and on said obtained physical data of said human body;
- mapping said plurality of images on the generated SSM-based 3D model of said human body target area, thereby obtaining an anatomical alignment of said plurality of images of said human body target area.

2. The method according to claim 1, wherein the first camera is one of an infrared camera, a UV camera or a visual light camera.

3. The method according to any of the preceding claims, wherein said first camera is configured to capture 2D images of said human body target area.

4. The method according to any of the preceding claims, further comprising the step of obtaining a plurality of visual images of at least said human body target area of said human body captured by a second camera over time, wherein the capturing of said plurality of images by said second camera has been done substantially simultaneously with the capturing of said plurality of images by said first camera.

5. The method according to claim 4, wherein the second camera is an RGB camera.

6. The method according to any of the preceding claims 4 or 5, further comprising the step of skeletonizing the obtained visual images of at least said human body target area.

7. The method according to any of the preceding claims 4 - 6, wherein the mapping of said plurality of images on the generated 3D model is based on the obtained plurality of visual images, in particular the skeletonized visual images.

8. The method according to any of the preceding claims, further comprising the step of warping the generated 3D model of at least said human body target area.

9. The method according to any of the preceding claims 4 - 7 and claim 8, wherein the warping of the generated 3D model is based on the obtained plurality of visual images, in particular the skeletonized visual images.

10. The method according to any of the preceding claims including at least claim 4, further comprising the step of estimating a relative position of said first camera with respect to said second camera based on a virtual image of the generated SSM-based 3D model of the human body target area.

11. A system for processing a plurality of camera images of a human body target area over time comprising:
- a first camera configured to capture a plurality of images of at least said human body target area of a human body over time,
- a controller and a memory with computer program code configured to perform the method according to any of the preceding claims.

12. A system according to claim 11, further comprising a second camera configured to capture a plurality of visual images of at least said human body target area over time substantially simultaneously with the capturing of images by the first camera.

13. A controller comprising at least one processor and at least one memory including computer program code, the at least one memory and computer program code configured to, with the at least one processor, cause the controller to perform the methods according to any of the preceding claims 1 -10.

14. A computer program product comprising computer-executable instructions for performing the methods according to any of the preceding claims 1 - 10 when the program is run on a computer.

15. A computer readable storage medium comprising computer-executable instructions for performing the methods according to any of the preceding claims 1 - 10 when the program is run on a computer.
